# EUROPEAN PATENT SPECIFICATION

(11) **EP 2 412 705 B1**
(45) Date of publication and mention of the grant of the patent: **07.05.2014**
(21) Application number: 10756236.5
(22) Date of filing: 26.03.2010
(51) Int. Cl.: C07D 207/333, A61K 31/40, A61P 1/16, A61P 3/08, A61P 5/14, A61P 5/18, A61P 9/00, A61P 9/04, A61P 9/10, A61P 13/12, A61P 19/08, A61P 21/04, A61P 25/00, A61P 25/08, A61P 25/28, A61P 25/32, A61P 31/04, A61P 35/00, A61P 43/00

(54) **NOVEL THERAPEUTIC AGENT FOR COGNITIVE IMPAIRMENT**
NEUES THERAPEUTIKUM FÜR KOGNITIVE BEEINTRÄCHTIGUNG
NOUVEL AGENT THÉRAPEUTIQUE POUR LE DÉFICIT COGNITIF

(30) Priority: 26.03.2009 JP 2009077120
(43) Date of publication of application: 01.02.2012
(73) Proprietor: Dainippon Sumitomo Pharma Co., Ltd., Osaka 541-8524 (JP)
(72) Inventor: KAKUYAMA, Hiroyoshi, Suita-shi Osaka 564-0053 (JP); SAKODA, Keiko, Suita-shi Osaka 564-0053 (JP); IWAMURA, Yoshihiro, Suita-shi Osaka 564-0053 (JP); IKEDA, Atsushi, Suita-shi Osaka 564-0053 (JP); TSUCHIDA, Atsushi, Osaka-shi Osaka 554-0022 (JP)
(74) Representative: Vossius & Partner
(86) International application number: PCT/JP2010/055419
(87) International publication number: WO 2010/110440

(56) References cited:
- EP-A1- 1 647 546
- WO-A1-00/32190
- WO-A1-2005/012245
- WO-A1-2007/145570
- WO-A1-2008/002539
- JP-T- 2002 536 294
- JP-T- 2004 516 313
- JP-T- 2005 501 817
- JP-T- 2007 519 759
- JP-T- 2007 527 918

## Description

The present invention relates to a novel medicament and pharmaceutical composition for use in preventing and/or treating cognitive impairment, in particular dementia. In more detail, the present invention relates to a novel medicament and pharmaceutical composition for use in preventing and/or treating cognitive impairment, in particular Alzheimer's dementia, which is characterized by an excellent brain penetration, an effect for lowering amyloid β and improving cognitive function, and high safety.

Alzheimer's dementia is a progressive neurodegenerative disease including various symptoms such as neurofibrillary tangle, senile plaque, neuronal atrophy, dendrite pruning and neuronal death. A conventional strategy for treating Alzheimer's dementia has been carried out by supplying neurotransmitters to patients thereof, which may alleviate the symptoms for a short period, but cannot stop the progression of neurodegeneration and neuronal death. Thus, it is necessary to ameliorate inflammation and neuronal death caused by neurodegeneration of Alzheimer's dementia, as well as a treatment alleviating the symptoms for a long period.

Fibrillization and deposition of amyloid β are key pathogenetic events of Alzheimer's dementia and precede other symptoms thereof. Hence, there have been attempts to produce a medicament for treating Alzheimer's dementia which can lower brain amyloid β as a functional mechanism. However, there is still no pharmaceutical agent which has a disease-modifying effect for Alzheimer's dementia.

Currently, amyloid β production inhibitors and amyloid β immunotherapies are expected to provide a disease-modifying effect for Alzheimer's dementia, having several mechanisms of action. However, for example, a γ secretase inhibitor which is known as an amyloid β production inhibitor inhibits enzymes as a functional mechanism, and thus γ secretase inhibitor is more likely to cause side effects by inhibiting other cleavages of substrates *in vivo.*

Namely, γ secretase is involved in the cleavage of proteins other than amyloid β such as Notch. For example, it is reported that when a γ secretase inhibitor is administered to a normal mouse, Notch signaling is inhibited and the mouse is affected with disorders of hematopoietic system and intestinal mucosa (Non-patent Reference 1).

The amyloid β immunotherapy is expected to ameliorate neuropathological symptoms in Alzheimer's dementia patients through the mechanism that microglial acts on Fc receptor to induce the phagocytosis of amyloid β. However, it is reported that the amyloid β immunotherapy causes meningoencephalitis as a side effect in 6 % of the patients (Non-patent Reference 2). In addition, it is also reported that immunotherapies inhibit the development of Alzheimer's dementia except for meningoencephalitis (Non-patent Reference 3). Therefore, a medicament having an action to enhance microglial phagocytosis of amyloid β is expected as a medicament for treating Alzheimer's dementia.

Recently, it is reported that the risk of onset of Alzheimer's dementia for diabetic patients is about twice as high as that of non-diabetic patients (Non-patent Reference 4).

Furthermore, it is reported that PPAR-γ agonists slow the progression of Alzheimer's dementia by suppressing inflammatory cytokines and/or β secretase, suppressing the decrease of IDE (insulin-degrading enzyme) which degrades amyloid β, and enhancing the clearance of amyloid β (Non-patent Reference 5).

Furthermore, a clinical study was conducted by administering rosiglitazone, a known PPAR-γ agonist, for 6 months to a patient with mild to moderate Alzheimer's dementia. The result thereof shows that high dose treatment (i.e. 8 mg) of rosiglitazone improved cognitive funtion in non-ApoE4 carrier patients (Non-patent Reference 6).

On the other hand, the post-marketing surveillance study shows that the frequency of fluid retention (peripheral edema) caused by rosiglitazone is high. Furthermore, it has been pointed out that rosiglitazone increases the risk of heart attack such as congestive heart failure, and causes side effects such as an onset or progression of diabetic macular edema (Non-patent Reference 7).

Besides the above-mentioned PPAR agonists, it has also been reported that derivatives having a heteroaryl structure exhibit PPAR-α/γ activating action (Patent Reference 1).

Furthermore, WO 00/32190 relates to a method for treating a subject, comprising administering a therapeutically effective amount of a PPARy agonist to said subject, wherein said subject is selected from subjects suffering from Alzheimer's disease, subjects susceptible to Alzheimer's disease, subjects suffering from a disease with an inflammatory component, and subjects susceptible to a disease with an inflammatory component.

[Patent Reference 1] WO 2005/012245
[Non-patent Reference 1] Wong G.T. et al: J. Biological. Chemistry, 279, 13, 12876, 2004
[Non-patent Reference 2] Schenk D. et al: NatRev. Neurosci., 3, 824, 2002
[Non-patent Reference 3] Hock C. et al: Neuron, 38, 547, 2003
[Non-patent Reference 4] Ott A. et al: Neurology, 53, 1937, 1999
[Non-patent Reference 5] Jiang Q. et al: CNS Drugs, 22, 1, 2008
[Non-patent Reference 6] Risner M.E. et al: Pharmacogenomics J., 6, 246, 2006
[Non-patent Reference 7] Singh S. & Loke Y.K.: Expert Opin. Drug Saf., 7, 579, 2008

The present invention provides a medicament and a pharmaceutical composition which are useful for preventing and/or treating cognitive impairment, specifically dementia, and more specifically Alzheimer's dementia, without causing side effects.

The present inventors extensively studied to find out a novel medicament for treating cognitive impairment, and then have found that the heteroaryl derivative disclosed in

Patent Reference 1 which exhibits PPAR-α/γ activating action has excellent properties of both amyloid-β-lowering effect and cognitive-function-improving effect, and induces little side effects. As these new findings are very useful to be a practical medicament, the present invention has been completed by it.

In particular, the present invention is as follows:
[1] A medicament or pharmaceutical composition for use in preventing and/or treating cognitive impairment comprising 2-methyl-2-[(4-{(1E)-3-[2-(4-methylbenzoyl)-1H-pyrrol-1-yl]prop-1-en-1-yl}benzyl)oxy]propionic acid; or a pharmaceutically acceptable salt or solvate thereof.
[2] The medicament or pharmaceutical composition for use according to [1] wherein the cognitive impairment is neurodegenerative cognitive impairment, vascular dementia, exogenous cognitive impairment, or mixed dementia thereof.
[3] The medicament or pharmaceutical composition for use according to [1] wherein the cognitive impairment is Alzheimer's dementia.
[4] The medicament or pharmaceutical composition for use according to [1] wherein the cognitive impairment is mixed dementia comprising Alzheimer's dementia and vascular dementia.
[5] The medicament or pharmaceutical composition for use according to [1] wherein the cognitive impairment is non-Alzheimer's dementia.
[6] The medicament or pharmaceutical composition for use according to [1] wherein the cognitive impairment is accompanied by schizophrenia, depression, bipolar depression, diabetes, attention deficit hyperactivity disorder, Creutzfeldt-Jakob disease, Kraepelin disease, Hallervorden-Spatz disease, spinocerebellar ataxia, progressive myoclonus epilepsy, progressive supranuclear palsy, viscous edema, parathyroid disease, Wilson disease, hepatic disease, hypoglycemia, remote symptoms of cancer, Cushing syndrome, uremia, arteriosclerosis, cerebral arteriosclerosis, chronic cerebral circulatory insufficiency, intracerebral hemorrhage, cerebral infarction, cerebral embolism, subarachnoid hemorrhage, chronic subdural hemorrhage, pseudobulbar palsy, aortic arch syndrome, Binswanger disease, arteriovenous malformation - thromboangiitis obliterans, hypoxia, anoxia, normal pressure hydrocephalus, Wernicke-Korsakoff syndrome, pellagra, Marchiafava-Bignami disease, vitamin B12 deficiency, disorders caused by metals, organic compounds, carbon monoxide, toxicants or drugs, brain tumor, open and closed head injury, Banti syndrome, fever attack, infection, bacterial meningitis, fungal meningitis, encephalitis, progressive multifocal leukoencephalopathy, Behcet syndrome, Kuru disease, syphilis, multiple sclerosis, muscular dystrophy, Whipple disease, concentration camp syndrome, disseminated lupus erythematosus, cardiac arrest, AIDS encephalopathy, hypothyroidism, hypopituitarism, or chronic alcoholism.
[7] The medicament or pharmaceutical composition for use according to [1] wherein the cognitive impairment is mild cognitive impairment.
[8] The medicament or pharmaceutical composition for use according to [1] which further comprises at least one additional medicament for preventing and/or treating cognitive impairment as a single formulation.
[9] The medicament or pharmaceutical composition for use according to [8] wherein the additional medicament for preventing and/or treating cognitive impairment is selected from the group consisting of acetylcholinesterase inhibitors, NMDA receptor antagonists, cyclooxygenase-2 selective inhibitors, intravenous human immunoglobulin formulations, 5-HT6 receptor antagonists, LTB4 receptor antagonists, human monoclonal antibodies, and amyloid β production inhibitors.
[10] The medicament or pharmaceutical composition for use according to [1] comprising a combination of 2-methyl-2-[(4-{(1E)-3-[2-(4-methylbenzoyl)-1H-pyrrol-1-yl]prop-1-en-1-yl}benzyl)oxy]-propionic acid or a pharmaceutically acceptable salt or solvate thereof, and at least one additional medicament for preventing and/or treating cognitive impairment.
[11] The medicament or pharmaceutical composition for use according to [10] wherein the additional medicament for preventing and/or treating cognitive impairment is selected from the group consisting of acetylcholinesterase inhibitors, NMDA receptor antagonists, cyclooxygenase-2 selective inhibitors, intravenous human immunoglobulin formulations, 5-HT6 receptor antagonists, LTB4 receptor antagonists, human monoclonal antibodies, and amyloid β production inhibitors.
[12] The medicament or pharmaceutical composition for use according to [10] wherein the 2-methyl-2-[(4-{(1E)-3-[2-(4-methylbenzoyl)-1H-pyrrol-1-yl]prop-1-en-1-yl}benzyl)oxy]-propionic acid or a pharmaceutically acceptable salt or solvate thereof is administered simultaneously or sequentially with the additional medicament for preventing and/or treating cognitive impairment.

The compound of the present invention has an excellent effect for improving cognitive function, and also has a disease-modifying effect for decreasing the amount of amyloid β which is perceived as a causative substance of Alzheimer's dementia. Consequently, the compound of the present invention is useful as a novel medicament for treating Alzheimer's dementia which is different from preexisting symptomatic treatment agents such as acetylcholinesterase inhibitors.

In particular, the inventors have found that the present compound has higher brain penetration compared with other clinically-used PPAR-γ agonists for treating diabetes, through Example 1 studying the ratio of concentrations in brain and peripheral plasma in a mouse.

In addition, it is known that clinically-used PPAR-γ agonists (e.g. rosiglitazone and pioglitazone) frequently cause fluid retention to occur side effects such as peripheral edema. In order to monitor the fluid retention caused by PPAR-γ agonists, hematocrit level is usually used as one of the indicators. In Example 5, the hematocrit level was decreased when rosiglitazone or pioglitazone was administered to mice, while the compound of the present invention did not significantly affect the hematocrit level. Hence, the compound of the present invention is different from the clinically-used PPAR-γ agonists, which can be expected as a highly safe medicament with little side effects. In other words, the compound of the present invention can be expected to be safely administered to Alzheimer's dementia patients for a long period.

Furthermore, the amounts of amyloid β 1-42 and amyloid β 1-40 in brain were decreased by administering the present compound to a transgenic mouse expressing APP mutation (i.e. mouse model of Alzheimer's dementia) in Example 2, which suggests that the present compound exhibits amyloid-β-lowering effect in brain. Moreover, the compound of the present invention exhibited the effect for increasing scavenger receptor CD36 which is involved in the action mechanism of phagocytosis of amyloid β in Example 3 using a primary microglia. Hence, it was found that the compound of the present invention has a pharmacological effect for decreasing the amount of brain amyloid β through the phagocytosis and degradation.

Furthermore, the percentage of freezing behavior was increased by administering the present compound to a transgenic mouse expressing APP mutation (i.e. mouse model of Alzheimer's dementia) in Example 4-1, which suggests that the present compound improved cognitive function. In addition, the compound of the present invention also improved cognitive function in a dose-dependent manner in an animal model of Alzheimer's dementia based on neurologic dysfunction caused by inflammation [e.g. cognitive impairment model induced by an inflammatory substance such as lipopolysaccharide (i.e. LPS)] in Example 4-2. These results show that the compound of the present invention is likely to have a different action mechanism from preexisting anti-dementia agents (e.g. acetylcholinesterase inhibitors). Therefore, the compound of the present invention can be useful as a novel medicament for treating dementia such as Alzheimer's dementia.

It is known that the amount of acetylcholine which is one of the neurotransmitters involved in cognitive function is decreased in the brain of Alzheimer's dementia patients. It is also known that scopolamine, which is a muscarinic acetylcholine receptor antagonist, causes memory impairment in animals and human. In the scopolamine-induced cognitive impairment rats, the compound of the present invention showed more potent improvement action of cognitive function compared with the preexisting PPAR-γ agonist, rosiglitazone.

Thus, the compound of the present invention has a high safety margin (i.e. few side effects), shows a better brain penetration than preexisting medicaments, and is expected to exhibit potent efficacy. Therefore, the compound of the present invention can be a novel medicament for treating Alzheimer's dementia.

In addition, the compound of the present invention can be used in combination with additional medicaments, for example, preexisting anti-dementia agents such as acetyl-cholinesterase inhibitors (e.g. donepezil) and memantine without departing from the purpose of the present invention.

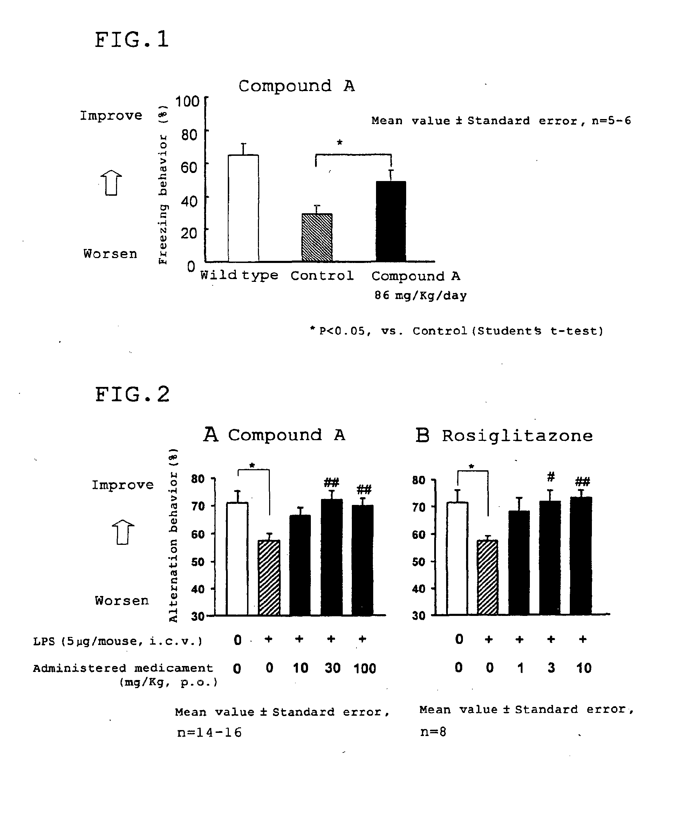

Figure 1 is a graph showing the effect for improving cognitive function by administering Compound A to a transgenic mouse expressing APP mutation, which was evaluated in a contextual fear conditioning test wherein the index of the evaluation was the increase of the percentage of freezing behavior (Example 4-1).

Figure 2 is a graph showing the effect for improving cognitive function in the Compound A treatment group (Figure 2A) and the rosiglitazone treatment group (Figure 2B), which was evaluated in a cognitive impairment model induced by an inflammatory substance wherein the index of the evaluation was the improvement of the percentage of alternation behavior.

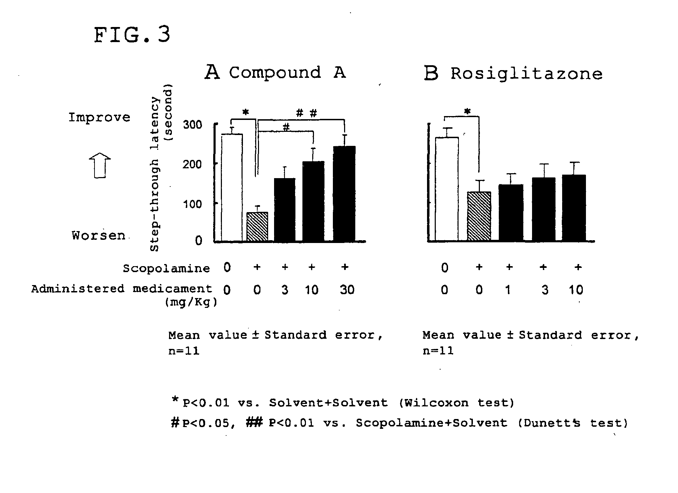

Figure 3 is a graph showing the effect improving cognitive function in the Compound A treatment group (Figure 3A) and the rosiglitazone treatment group (Figure 3B), which was evaluated in a scopolamine-induced cognitive impairment model wherein the index of the evaluation was the increase of step-through latency in passive avoidance response (second).

Hereinafter, the present invention is explained in more detail.

2-Methyl-2-[(4-{(1E)-3-[2-(4-methylbenzoyl)-1H-pyrrol-1-yl]prop-1-en-1-yl}benzyl)oxy]propionic acid (hereinafter, referred to as Compound A) is disclosed in WO 2005/012245 (Example 3B), and the chemical structure thereof is shown below. Compound A can be prepared by the process disclosed in WO 2005/012245. In addition, one or more hydrogen atoms of Compound A may be optionally substituted with deuterium.

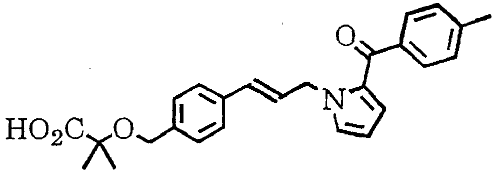

The pharmaceutically acceptable salt used herein includes, for example, salts with an organic base (e.g. diethanolamine salt, ethylenediamine salt and N-methylglucamine salt) and salts with an inorganic base (e.g. a salt with an alkali earth metal such as calcium salt and magnesium salt, and a salt with an alkali metal such as lithium salt, potassium salt and sodium salt).

In addition, the present invention includes hydrates of the present compound, and solvates of the present compound with an organic solvent (e.g. ethanol solvate). Furthermore, the present invention includes all types of crystal forms of the present compound.

When the compound of the present invention is used for preventing or treating the disease, the compound can be administered orally or parenterally (e.g. intravenously, subcutaneously, intramuscularly, locally, transrectally, percutaneously, intraspinally and nasally) in a drug formulation (e.g. pharmaceutical composition). The oral formulation includes, for example, tablets, capsules, pills, granules, powders, liquids, and suspensions. The parenteral formulation includes, for example, injectable aqueous or oily solutions, ointments, creams, lotions, aerosols, suppositories, and adhesive skin patches. These formulations can be prepared by well-known conventional techniques, and can comprise nontoxic and inert carriers or additives which are commonly used in medicinal field.

The dosage of the present compound may vary according to, for example, the form of the compound, and patient's disease, age, body weight, sex, symptom, and administration route. Typically, Compound A is administered in a dosage of 0.001-4000 mg/day, preferably 0.01-400 mg/day, more preferably 0.1-40 mg/day, and most preferably 0.5-20 mg/day for an adult (body weight 50 kg), and once to several times (e.g. twice to 3 times) a day. And, Compound A can be administered once in several days to several weeks.

The pharmaceutical composition comprising the present compound may further comprise preexisting medicaments for preventing or treating cognitive impairments as a single formulation unless the purpose of the present invention fails. Furthermore, the present compound can be administered simultaneously or sequentially in combination with other preexisting medicament(s) for preventing or treating cognitive impairments unless the purpose of the present invention fails. The other preexisting medicaments for preventing or treating cognitive impairments include, for example, acetylcholinesterase inhibitors (e.g. donepezil, galantamine, rivastigmine and tacrine) and NMDA receptor antagonists (e.g. memantine and neramexane). The other examples thereof also include, but are not limited to, cyclooxygenase-2 selective inhibitors (e.g. celecoxib), intravenous human immunoglobulin formulations (e.g. gammagard), 5-HT6 receptor antagonists (e.g. PF-5212365), LTB4 receptor antagonists (e.g. ethyl icosapentate), human monoclonal antibodies (e.g. bapineuzumab and solanezumab), and amyloid β production inhibitors (e.g. semagacestat and BMS-708163).

The term "cognitive impairment" means a deterioration or loss of mental function caused mainly by acquired organic brain lesions, but various diseases may be involved in the primary disease of cognitive impairment. Cognitive impairment is categorized as vascular dementia, neurodegenerative cognitive impairment, and exogenous cognitive impairment based on the causes thereof. These diseases may occur simultaneously, which is referred to as mixed dementia. In particular, the mixed dementia may be caused by the below-mentioned Alzheimer's dementia and vascular dementia. Cognitive impairment also includes mild cognitive impairment (MCI), i.e. a prior stage in which the symptom may develop to various types of dementia.

Vascular dementia means dementia caused by cerebrovascular disorder, cerebral infarction or intracerebral hemorrhage.

Representative examples of neurodegenerative cognitive impairment include Alzheimer's dementia and non-Alzheimer's dementia.

It is known that non-Alzheimer's dementia includes, for example, dementia with Lewy body, neurofibrillary tangle dementia, Parkinson disease, Huntington disease, frontotemporal dementia [Pick disease, progressive subcortical gliosis (PSG), amyotrophic lateral sclerosis with dementia (ALS-D), frontal lobe dementia, frontotemporal dementia and parkinsonism linked to chromosome 17 (FTDP-17)], dementia with glial tangles [progressive supranuclear palsy (PSP), corticobasal degeneration (CBD)], argyrophilic grain disease, dementias with predominant degeneration in subcortical nucleus [Huntington disease, dentatorubral-pallidoluysian atrophy (DRPLA), thalamic degeneration], and other types of degenerative dementia difficult to be classified.

Exogenous cognitive impairment includes diseases caused by, for example, schizophrenia, depression, bipolar depression, diabetes, attention deficit hyperactivity disorder, Creutzfeldt-Jakob disease, Kraepelin disease, Hallervorden-Spatz disease, spinocerebellar ataxia, progressive myoclonus epilepsy, progressive supranuclear palsy, viscous edema, parathyroid disease, Wilson disease, hepatic disease, hypoglycemia, remote symptoms of cancer, Cushing syndrome, uremia, arteriosclerosis, cerebral arteriosclerosis, chronic cerebral circulatory insufficiency, intracerebral hemorrhage, cerebral infarction, cerebral embolism, subarachnoid hemorrhage, chronic subdural hemorrhage, pseudobulbar palsy, aortic arch syndrome, Binswanger disease, arteriovenous malformation - thromboangiitis obliterans, hypoxia, anoxia, normal pressure hydrocephalus, Wernicke-Korsakoff syndrome, pellagra, Marchiafava-Bignami disease, vitamin B12 deficiency, disorders caused by metals, organic compounds, carbon monoxide, toxicants or drugs, brain tumor, open and closed head injury, Banti syndrome, fever attack, infection, bacterial meningitis, fungal meningitis, encephalitis, progressive multifocal leukoencephalopathy, Behcet syndrome, Kuru disease, syphilis, multiple sclerosis, muscular dystrophy, Whipple disease, concentration camp syndrome, disseminated lupus erythematosus, cardiac arrest, AIDS encephalopathy, hypothyroidism, hypopituitarism, and chronic alcoholism.

### Example

The present invention is illustrated in more detail by the following Reference Examples, Examples and Tests, but should not be construed to be limited thereto. In addition, the compound names of the following examples do not necessarily correspond to the IUPAC nomenclature. Some terms may be defined by abbreviations for the sake of conciseness, which are as defined above. In particular, the compound of the present invention is abbreviated as Compound A.

### Example 1 (Brain delivery property)

Method: Compound A or rosiglitazone (10 mg/kg) was orally administered in one portion to a mouse (C57BL/6J: female, 11 weeks old, 3 mice at each measuring point) under non-fasting condition. Its brain tissue and plasma were sequentially sampled for 6 hours after the administration to measure the concentration of the administered-compound. Based on each mean value of the concentrations in its brain tissue and plasma measured at several time points, each elimination half-life (t_{1/2}) and area under the curve (AUC₀₋₆ₕᵣ) of the administered-compound were calculated.

Result: With regard to AUC₀₋₆ₕᵣ ratio in brain/plasma (Kp value) which indicates brain penetration of each compound after the administration, Kp value of Compound A was 0.154 and that of rosiglitazone was 0.0451, i.e., Kp value of Compound A was 3.4 times higher than that of rosiglitazone. This result shows that brain-delivery property of Compound A was higher than that of rosiglitazone. And, in both the compounds, t_{1/2} was not so different between brain tissue and plasma, and hence the brain concentration seemed to decrease in parallel with the plasma concentration (Table 1).

**Table 1**

| Administered medicament | Tissue | t_{1/2} (hr) | AUC₀₋₆ₕᵣ (ug·hr/mL) | Kp value (brain/plasma AUC₀₋₆ₕᵣ ratio) |
|---|---|---|---|---|
| Compound A | Plasma | 4.59 | 9.71 | - |
| | Brain | 4.26 | 1.50 | 0.154 |
| Rosiglitazone | Plasma | 1.26 | 40.4 | - |
| | Brain | 1.06 | 1.82 | 0.0451 |

### Example 2 (Amyloid-β-lowering effect in brain)

A mixture of Compound A (86 mg/kg/day) and feeds was administered for 10 weeks to a transgenic mouse expressing APP Swedish mutation (15-16 months old, female, Taconic Farms, Inc.). Then, its brain was removed to measure the amounts of guanidine-soluble amyloid β in cerebral cortex and hippocampus by ELISA (the measured amount is perceived as a total amount of amyloid β).

**Table 2**

| | Total amount of amyloid β 1-40 (p mol/g brain) | | Total amount of amyloid β 1-42 (p mol/g brain) | |
|---|---|---|---|---|
| Administered medicament | Hippocampus | Cerebral cortex | Hippocampus | Cerebral cortex |
| Control | 3161 ± 746 | 46035 ± 28844 | 949 ± 321 | 11067 ± 5028 |
| Compound A 86 mg/kg/day | 3208 ± 1932 | 26589 ± 7554 | 732 ± 354 | 8746 ± 2216 |

| | | | | |
|---|---|---|---|---|
| Mean value ± Standard error (n=5-6) | | | | |

It is known that the amount of amyloid β in brain is increased in Alzheimer's dementia patients, and thus amyloid β is perceived as a causative substance of Alzheimer's dementia. It was found that Compound A lowered the amount of amyloid β 1-40 and amyloid β 1-42 which are main ingredients of amyloid β in brain (i.e. hippocampus and cerebral cortex). In particular, Compound A lowered the amount of amyloid β 1-42 in both hippocampus and cerebral cortex, which shows a particularly high neurotoxicity (Table 2). It suggests that Compound A can slow the progression of Alzheimer's dementia.

### Example 3 (Mechanism of action for lowering amyloid β) [0052]

Microglial cells derived from neonatal rats (Sumitomo Bakelite) were cultured for 24 hours in a culture medium containing Compound A (0.5 µM or 5 µM). Then, total RNA was extracted from the cells, and therewith the transcription levels of scavenger receptor CD36 and glyceraldehyde-3-phosphate dehydrogenase (GAPDH) were measured by real-time RT-PCR to calculate the CD36 transcription level as a value relative to the GAPDH transcription level. In order to compare the CD36 transcription levels treated in the different conditions, CD36 transcription level in the control that was cultured for 24 hours with only DMSO which was used as a solvent in the above cultivation of Compound A was measured, and then the relative ratio of each CD36 transcription level of Compound A per that of the control (=1) was calculated.

The results showed that 5 µM Compound A induced the increase of the CD36 transcription level (Table 3).

Since the amount of brain amyloid β increases as Alzheimer's dementia progresses, the results of Example 3 indicated that Compound A can slow the progression of Alzheimer's dementia by enhancing microglial phagocytosis and degradation of amyloid β.

**Table 3**

| Treated condition | 24 hours |
|---|---|
| DMSO | 1 |
| Compound A 0.5 µM | 1.44 |
| Compound A 5 µM | 1.99 |

### Example 4-1 (Effect for improving cognitive function in transgenic mouse expressing APP Swedish mutation)

A mixture of Compound A (86 mg/kg/day) and feeds was administered for 10 weeks to a transgenic mouse expressing APP Swedish mutation (15-16 months old, female, Taconic Farms, Inc.) which is the same type as used in Example 2. Then, the contextual fear conditioning test (Proc. Natl. Acad. Sci., USA, 104, 5161, 2006) was performed in order to evaluate cognitive function thereof using freezing behavior as an index. When a statistically-significant increase of the freezing behavior (%) was observed, the result was judged to exhibit an effect for improving cognitive function. As a result, the cognitive function in the control group using the transgenic mouse expressing APP Swedish mutation was significantly lowered, compared with the result using a wild-type mouse. However, the cognitive function in the Compound A treatment group was significantly improved, compared with the control group (Table 4 and Figure 1).

**Table 4**

| | Administered medicament | Freezing behavior (%) |
|---|---|---|
| Wild type | - | 66.4 ± 8.3 |
| Transgenic mouse expressing APP Swedish mutation | Control | 28.8 ± 4.8 |
| | Compound A 86 mg/Kg/day | 48.6 ± 6.2* |

| | | |
|---|---|---|
| * P<0.05, vs. Control (Student's t-test, two- sided significance level 5 %) Mean value ± Standard error (n=5-6) | | |

### Example 4-2 (Effect for improving cognitive function in cognitive impairment model induced by inflammatory substance)

It is known that lipopolysaccharide (LPS) can experimentally induce an inflammatory condition in brain like in Alzheimer's dementia patients. Thus, LPS-induced model is thought as a good disease model to study cognitive impairment caused by inflammation in Alzheimer's dementia (Neurosci. Res., 61, 113, 2008).

In Example 4-2, 5 µg of LPS (*Eschenchia coli* 055:B5, SIGMA) was intracerebroventricularly administered to a mouse (Slc:ddY mouse, 4 weeks old, male, Japan SLC, Inc.) in both ventricles, i.e. 2.5 µl of 1 mg/ml LPS was administered to each ventricle. LPS induced cognitive impairment in Y-maze test was appeared 4 days after treatment. (i.e. decrease of alternation behavior). Compound A (10, 30, 100 mg/kg, p.o., n=14-16) or rosiglitazone (1, 3, 10 mg/kg, p.o., n=8) was administered 30 minutes before the LPS administration, and 1, 2, 3 days after the LPS administration, and also 1 hour before the Y-maze test to evaluate the effect for improving cognitive function. 0.5 % Methylcellulose solution was used as a solvent of each compound.

When a statistically-significant increase of the the percentage of alternation behavior was observed, the result was judged to exhibit an effect for improving cognitive function.

As a result, the alternation behavior in the Compound A treatment group was significantly improved at doses of 30 mg/kg and 100 mg/kg (Table 5 and Figure 2A).

In the rosiglitazone treatment group, the alternation behavior was significantly improved at doses of 3 mg/kg and 10 mg/kg (Table 6 and Figure 2B).

**Table 5**

| Administered medicament | | Alternation behavior |
|---|---|---|
| i.c.v. | p.o. | |
| Solvent | Solvent | 70.9 ± 4.3 |
| LPS | Solvent | 57.5 ± 2.5 * |
| LPS | Compound A 10 mg/Kg | 66.5 ± 2.8 |
| LPS | Compound A 30 mg/Kg | 72.2 ± 3.2 ## |
| LPS | Compound A 100 mg/Kg | 69.9 ± 2.9 ## |

| | | |
|---|---|---|
| * P<0.05 vs. Solvent + Solvent (Wilcoxon test) ## P<0.01 vs. LPS + Solvent (Dunnett's test) | | |

**Table 6**

| Administered medicament | | Alternation behavior |
|---|---|---|
| i.c.v. | p.o. | |
| Solvent | Solvent | 71.5 ± 4.5 |
| LPS | Solvent | 57.5 ± 1.8 * |
| LPS | Rosiglitazone 1 mg/kg | 68.4 ± 5.0 |
| LPS | Rosiglitazone 3 mg/kg | 71.9 ± 4.1 # |
| LPS | Rosiglitazone 10 mg/kg | 73.4 ± 2.7 ## |

| | | |
|---|---|---|
| * P<0.05 vs. Solvent + Solvent (Wilcoxon test) # P<0.05, ## P<0.01 vs. LPS + Solvent (Dunnett's test) | | |

### Example 5 (Measuring fluid retention as side effect)

High-fat diet (D12492, Research Diet Inc.) was given to a mouse (C57BL/6J, male, 4 weeks old, CLEA Japan) for 26 weeks to induce obese diabetes. And, rosiglitazone (0.1, 0.3, 1, 3, 10 mg/kg, n=8) or Compound A (3, 10, 30, 100 mg/kg, n=8) was orally administered once a day for 8 weeks to the mice, then hematocrit level was measured as an index of side effect (fluid retention) (Tables 7 and 8). 0.5 % Methylcellulose solution was used as a solvent for each compound.

As a result, the hematocrit level in the rosiglitazone treatment group was significantly decreased at a dose of 0.3 mg/kg or more.

On the other hand, the hematocrit level in the Compound A treatment group was not significantly changed even at a dose of 100 mg/kg which was the highest dose tested in Example 5 (Table 8).

Consequently, Compound A did not exhibit any side effect (fluid retention) though rosiglitazone exhibited it. In other words, Compound A is expected to be superior to preexisting medicaments in clinical safety-margin because Compound A does not affect hematocrit level at a dose around the pharmaceutically effective amount.

**Table 7**

| Administered medicament | Hematocrit level (%) |
|---|---|
| Solvent | 48.3 ± 0.9 |
| Rosiglitazone 0.1 mg/kg | 47.4 ± 1.9 |
| Rosiglitazone 0.3 mg/kg | 46.6 ± 1.9 ^{#} |
| Rosiglitazone 1 mg/kg | 45.6 ± 0.9 ^{##} |
| Rosiglitazone 3 mg/kg | 94.7 ± 1.5 ^{##} |
| Rosiglitazone 10 mg/kg | 41.1 ± 4.9 ^{##} |

| | |
|---|---|
| # P<0.05, ## P<0.01 vs. Solvent (Student's t-test) | |

**Table 8**

| Administered medicament | Hematocrit level (%) |
|---|---|
| Solvent | 47.4 ± 1.3 |
| Compound A 3 mg/kg | 47.7 ± 2.0 |
| Compound A 10 mg/kg | 48.7 ± 1.2 |
| Compound A 30 mg/kg | 48.7 ± 1.5 |
| Compound A 100 mg/kg | 48.4 ± 1.6 |

| | |
|---|---|
| Hematocrit level did not significantly differ between the solvent-administered group and the Compound-A-administered groups (Student's t-test). | |

### Example 6 (Effect for improving Cognitive function in scopolamine-induced cognitive impairment model)

It is known that scopolamine is a muscarinic acetylcholine receptor antagonist which causes memory impairment in animals and human. Thus, in order to evaluate the efficacy of an anti-Alzheimer's drug, a scopolamine-induced cognitive-impairment model is used in many evaluation tests of cognitive function including a passive avoidance test (Eur. J. Pharmacol., 383, 231, 1999). In Example 6, the effect of Compound A and rosiglitazone on cognitive function was studied using a passive avoidance test using rats with scopolamine-induced cognitive impairment.

Compound A (3, 10, 30 mg/kg) or rosiglitazone (1, 3, 10 mg/kg) was orally administered to a rat 60 minutes before training for the passive avoidance test. Scopolamine (0.5 mg/kg) or saline was intraperitoneally administered 30 minutes before the training. In the training period, the rat was put in a bright compartment and was allowed to explore for 10 seconds. Then, a door between the bright compartment and the dark one was opened, and as the rat went into the dark compartment, the door was closed. Three seconds later, the rat was given electroconvulsive shock (0.5 mA, 3 seconds). The passive avoidance response test was carried out 24 hours after the training. The rat was put in the bright compartment and was monitored to record the time that the rat stayed in the bright compartment (step-through latency) for up to 300 seconds. Dunnett's multiple comparison test (two-sided significance level 5 %) was carried out to compare the step-through latency between the scopolamine treatment group and the scopolamine + test medicament treatment group. When a significant increase of the step-through latency (second) was observed, the result was judged to exhibit an effect for improving cognitive function. 0.5 % Methylcellulose solution was used as a solvent of each compound.

The single oral administration of Compound A (3-30mg/kg) improved cognitive function in a dose-dependent manner and in a statistically-significant manner at 10 mg/kg and 30 mg/kg (Table 9 and Figure 3). On the other hand, rosiglitazone (1-10 mg/kg, p.o.) did not increase step-thorough latency (second) with statistically-significance (Table 10 and Figure 3). Thus, it was shown that the single oral administration of Compound A has a stronger effect for improving cognitive function than the preexisting PPAR-γ agonist, rosiglitazone.

**Table 9**

| Administered medicament | | Step-through latency (second) mean value ± standard error, n=11 |
|---|---|---|
| i.p. | p.o. | |
| Solvent | Solvent | 272.5 + 18.6 |
| Scopolamine | Solvent | 75.4 ± 17.4 * |
| Scopolamine | Compound A 3 mg/kg | 162.5 ± 28.2 |
| Scopolamine | Compound A 10 mg/kg | 204.2 ± 32.5 # |
| Scopolamine | Compound A 30 mg/kg | 242.2 ± 28.8 ## |

| | | |
|---|---|---|
| * P<0.05 vs. Solvent + Solvent (Wilcoxon test) # P<0.05, ## P<0.01 vs. Scopolamine + Solvent (Dunnett's test) | | |

**Table 10**

| Administered medicament | | Step-through latency (second) mean value ± standard error, n=11 |
|---|---|---|
| i.p. | p.o. | |
| Solvent | Solvent | 263.2 ± 26.3 |
| Scopolamine | Solvent | 128.5 ± 27.9* |
| Scopolamine | Rosiglitazone mg/kg | 144.8 ± 28.3 |
| Scopolamine | Rosiglitazone 3 mg/kg | 164.5 ± 33.1 |
| Scopolamine | Rosiglitazone 10 mg/kg | 170.7 ± 30.2 |

| | | |
|---|---|---|
| * P<0.01 vs. Solvent Solvent (Wilcoxon test) | | |

The compound of the present invention is expected to exhibit excellent effects for lowering amyloid β in brain and improving cognitive function at clinical practice. Furthermore, using the present compound, the present invention can provide a very safe medicament and pharmaceutical composition for use in preventing and/or treating cognitive impairment, especially Alzheimer's dementia.

## Claims

1. A medicament or pharmaceutical composition for use in preventing and/or treating cognitive impairment comprising 2-methyl-2-[(4-{(1E)-3-[2-(4-methylbenzoyl)-1H-pyrrol-1-yl]prop-1-en-1-yl}benzyl)oxy]propionic acid, or a pharmaceutically acceptable salt or solvate thereof.

2. The medicament or pharmaceutical composition for use according to claim 1 wherein the cognitive impairment is neurodegenerative cognitive impairment, vascular dementia, exogenous cognitive impairment, or mixed dementia thereof.

3. The medicament or pharmaceutical composition for use according to claim 1 wherein the cognitive impairment is Alzheimer's dementia.

4. The medicament or pharmaceutical composition for use according to claim 1 wherein the cognitive impairment is mixed dementia comprising Alzheimer's dementia and vascular dementia.

5. The medicament or pharmaceutical composition for use according to claim 1 wherein the cognitive impairment is non-Alzheimer's dementia.

6. The medicament or pharmaceutical composition for use according to claim 1 wherein the cognitive impairment is accompanied by schizophrenia, depression, bipolar depression, diabetes, attention deficit hyperactivity disorder, Creutzfeldt-Jakob disease, Kraepelin disease, Hallervorden-Spatz disease, spinocerebellar ataxia, progressive myoclonus epilepsy, progressive supranuclear palsy, viscous edema, parathyroid disease, Wilson disease, hepatic disease, hypoglycemia, remote symptoms of cancer, Cushing syndrome, uremia, arteriosclerosis, cerebral arteriosclerosis, chronic cerebral circulatory insufficiency, intracerebral hemorrhage, cerebral infarction, cerebral embolism, subarachnoid hemorrhage, chronic subdural hemorrhage, pseudobulbar palsy, aortic arch syndrome, Binswanger disease, arteriovenous malformation, thromboangiitis obliterans, hypoxia, anoxia, normal pressure hydrocephalus, Wernicke-Korsakoff syndrome, pellagra, Marchiafava-Bignami disease, vitamin B12 deficiency, disorders caused by metals, organic compounds, carbon monoxide, toxicants or drugs, brain tumor, open and closed head injury, Banti syndrome, fever attack, infection, bacterial meningitis, fungal meningitis, encephalitis, progressive multifocal leukoencephalopathy, Behcet syndrome, Kuru disease, syphilis, multiple sclerosis, muscular dystrophy, Whipple disease, concentration camp syndrome, disseminated lupus erythematosus, cardiac arrest, AIDS encephalopathy, hypothyroidism, hypopituitarism, or chronic alcoholism.

7. The medicament or pharmaceutical composition for use according to claim 1 wherein the cognitive impairment is mild cognitive impairment.

8. The medicament or pharmaceutical composition for use according to claim 1 which further comprises at least one additional medicament for preventing and/or treating cognitive impairment as a single formulation.

9. The medicament or pharmaceutical composition for use according to claim 8 wherein the additional medicament for preventing and/or treating cognitive impairment is selected from the group consisting of acetylcholinesterase inhibitors, NMDA receptor antagonists, cyclooxygenase-2 selective inhibitors, intravenous human immunoglobulin formulations, 5-HT6 receptor antagonists, LTB4 receptor antagonists, human monoclonal antibodies, and amyloid β production inhibitors.

10. The medicament or pharmaceutical composition for use according to claim 1 which comprises at least one additional medicament for preventing and/or treating cognitive impairment.

11. The medicament or pharmaceutical composition for use according to claim 10 wherein the additional medicament for preventing and/or treating cognitive impairment is selected from the group consisting of acetylcholinesterase inhibitors, NMDA receptor antagonists, cyclooxygenase-2 selective inhibitors, intravenous human immunoglobulin formulations, 5-HT6 receptor antagonists, LTB4 receptor antagonists, human monoclonal antibodies, and amyloid β production inhibitors.

12. The medicament or pharmaceutical composition for use according to claim 10 wherein the 2-methyl-2-[(4-{(E)-3-[2-(4-methylbenzoyl)-1H-pyrrol-1-yl]prop-1-en-1-yl}benzyl)oxy]propionic acid or a pharmaceutically acceptable salt or solvate thereof is administered simultaneously or sequentially with the additional medicament for preventing and/or treating cognitive impairment.

## Patentansprüche

1. Ein Medikament oder Arzneimittel zur Verwendung bei der Vorbeugung und/oder Behandlung kognitiver Beeinträchtigung, umfassend 2-Methyl-2-[(4-{(1E)-3-[2-(4-methylbenzoyl)-1H-pyrrol-1-yl]prop-1-en-1-yl}benzyl)oxy]propionsäure oder ein pharmazeutisch verträgliches Salz oder Solvat davon.

2. Das Medikament oder Arzneimittel zur Verwendung nach Anspruch 1, wobei es sich bei der kognitiven Beeinträchtigung um neurodegenerative kognitive Beeinträchtigung, vaskuläre Demenz, exogene kognitive Beeinträchtigung oder um eine gemischte Demenz davon handelt.

3. Das Medikament oder Arzneimittel zur Verwendung nach Anspruch 1, wobei es sich bei der kognitiven Beeinträchtigung um Alzheimer-Demenz handelt.

4. Das Medikament oder Arzneimittel zur Verwendung nach Anspruch 1, wobei es sich bei der kognitiven Beeinträchtigung um gemischte Demenz, umfassend Alzheimer-Demenz und vaskuläre Demenz, handelt.

5. Das Medikament oder Arzneimittel zur Verwendung nach Anspruch 1, wobei es sich bei der kognitiven Beeinträchtigung um Nicht-Alzheimer-Demenz handelt.

6. Das Medikament oder Arzneimittel zur Verwendung nach Anspruch 1, wobei die kognitive Beeinträchtigung von Schizophrenie, Depression, bipolarer Depression, Diabetes, Aufinerksamkeitsdefizit-Hyperaktivitätsstörung, Creutzfeldt-Jakob-Krankheit, Kraepelin-Krankheit, Hallervorden-Spatz-Krankheit, spinozerebellärer Ataxie, progressiver Myoklonusepilepsie, progressiver supranukleärer Blickparese, viskösem Ödem, Erkrankung der Nebenschilddrüsen, Wilson-Krankheit, hepatischer Erkrankung, Hypoglykämie, entfernten Symptomen von Krebs, Cushing-Syndrom, Urämie, Arteriosklerose, zerebraler Arteriosklerose, chronischer zerebraler Kreislaufinsuffizienz, intrazerebraler Hämorrhagie, zerebralem Infarkt, zerebraler Embolie, subarachnoidaler Hämorrhagie, chronischer subduraler Hämorrhagie, Pseudobulbärparalyse, Aortenbogensyndrom, Binswanger-Erkrankung, arteriovenöser Fehlbildung, Thrombangiitis obliterans, Hypoxie, Anoxie, Normaldruckhydrozephalus, Wernicke-Korsakoff-Syndrom, Pellagra, Marchiafava-Bignami-Krankheit, Vitamin-B12-Mangel, Störungen, die durch Metalle, organische Verbindungen, Kohlenstoffmonoxid, Toxine oder Arzneimittel ausgelöst werden, Gehirntumor, offener und geschlossener Kopfverletzung, Banti-Syndrom, Fieberattacke, Infektion, bakterieller Meningitis, fungaler Meningitis, Enzephalitis, progressiver multifokaler Leukoenzephalopathie, Behcet-Syndrom, Kuru-Krankheit, Syphilis, Multipler Sklerose, muskulärer Dystrophie, Whipple-Krankheit, Konzentrationslager-Syndrom, disseminierter Lupus erythematodes, Herzstillstand, AIDS-Enzephalopathie, Hypothyroidismus, Hypopituitarismus, oder chronischem Alkoholismus begleitet wird.

7. Das Medikament oder Arzneimittel zur Verwendung nach Anspruch 1, wobei es sich bei der kognitiven Beeinträchtigung um leichte kognitive Beeinträchtigung handelt.

8. Das Medikament oder Arzneimittel zur Verwendung nach Anspruch 1, welches ferner mindestens ein zusätzliches Medikament zur Vorbeugung und/oder Behandlung kognitiver Beeinträchtigung als eine Einzelformulierung umfasst.

9. Das Medikament oder Arzneimittel zur Verwendung nach Anspruch 8, wobei das zusätzliche Medikament zur Vorbeugung und/oder Behandlung kognitiver Beeinträchtigung aus der Gruppe bestehend aus Acetylcholinesteraseinhibitoren, NMDA-Rezeptorantagonisten, selektiven Cyclooxygenase-2-Inhibitoren, intravenösen Human-Immunglobulin-Formulierungen, 5-HT6-Rezeptorantagonisten, LTB4-Rezeptorantagonisten, humanen monoklonalen Antikörpern und Amyloid-β-Produktionsinhibitoren ausgewählt ist.

10. Das Medikament oder Arzneimittel zur Verwendung nach Anspruch 1, welches mindestens ein zusätzliches Medikament zur Vorbeugung und/oder Behandlung kognitiver Beeinträchtigung umfasst.

11. Das Medikament oder Arzneimittel zur Verwendung nach Anspruch 10, wobei das zusätzliche Medikament zur Vorbeugung und/oder Behandlung kognitiver Beeinträchtigung aus der Gruppe bestehend aus Acetylcholinesteraseinhibitoren, NMDA-Rezeptorantagonisten, selektiven Cyclooxygenase-2-Inhibitoren, intravenösen Human-Immunglobulin-Formulierungen, 5-HT6-Rezeptorantagonisten, LTB4-Rezeptorantagonisten, humanen monoklonalen Antikörpern und Amyloid-β-Produktionsinhibitoren ausgewählt ist.

12. Das Medikament oder Arzneimittel zur Verwendung nach Anspruch 10, wobei die 2-Methyl-2-[(4-{(1E)-3-[2-(4-methylbenzoyl)-1H-pyrrol-1-yl]prop-1-en-1-yl}benzyl)oxy]propionsäure oder ein pharmazeutisch verträgliches Salz oder Solvat davon simultan oder sequenziell mit dem zusätzlichen Medikament zur Vorbeugung und/oder Behandlung kognitiver Beeinträchtigung verabreicht wird.

## Revendications

1. Médicament ou composition pharmaceutique destiné à être utilisé dans la prévention et/ou le traitement d'une détérioration cognitive comprenant de l'acide 2-méthyl-2-[(4-{(1E)-3-[2-(4-méthylbenzoyl)-1H-pyrrol-1-yl]prop-1-én-1-yl}benzyl)oxy]propionique, ou un sel ou solvate pharmaceutiquement acceptable de celui-ci.

2. Médicament ou composition pharmaceutique destiné à être utilisé selon la revendication 1 où la détérioration cognitive est une détérioration cognitive neurodégénérative, la démence vasculaire, une détérioration cognitive exogène ou une démence mixte de celles-ci.

3. Médicament ou composition pharmaceutique destiné à être utilisé selon la revendication 1 où la détérioration cognitive est la démence d'Alzheimer.

4. Médicament ou composition pharmaceutique destiné à être utilisé selon la revendication 1 où la détérioration cognitive est une démence mixte comprenant la démence d'Alzheimer et la démence vasculaire.

5. Médicament ou composition pharmaceutique destiné à être utilisé selon la revendication 1 où la détérioration cognitive est une démence non-Alzheimer.

6. Médicament ou composition pharmaceutique destiné à être utilisé selon la revendication 1 où la détérioration cognitive est accompagnée par la schizophrénie, la dépression, la dépression bipolaire, le diabète, le trouble d'hyperactivité avec déficit de l'attention, la maladie de Creutzfeldt-Jakob, la maladie de Kraepelin, la maladie d'Hallervorden-Spatz, l'ataxie spinocérébelleuse, la myoclonie épileptique progressive, la paralysie supra-nucléaire progressive, l'oedème visqueux, la maladie parathyroïdienne, la maladie de Wilson, la maladie hépatique, l'hypoglycémie, les symptômes éloignés du cancer, le syndrome de Cushing, l'urémie, l'artériosclérose, l'artériosclérose cérébrale, l'insuffisance circulatoire cérébrale chronique, l'hémorragie intracérébrale, l'infarctus cérébral, l'embolie cérébrale, hémorragie sous-arachnoïdienne, l'hémorragie sous-durale chronique, la paralysie pseudobulbaire, le syndrome de la crosse aortique, la maladie de Binswanger, la malformation artérioveineuse, thromboangéite oblitérante, l'hypoxie, l'anoxie, l'hydrocéphalie à pression normale, le syndrome de Wernicke-Korsakoff, la pellagre, la maladie de Marchiafava-Bignami, la carence en vitamine B12, les troubles causés par les métaux, les composés organiques, le monoxyde de carbone, les substances toxiques ou les médicaments, la tumeur cérébrale, le traumatisme cranio-cérébral ouvert et fermé, le syndrome de Banti, l'accès de fièvre, l'infection, la méningite bactérienne, la méningite fongique, l'encéphalite, la leuco-encéphalopathie multifocale progressive, le syndrome de Behcet, la maladie de Kuru, la syphilis, la sclérose en plaques, la dystrophie musculaire, la maladie de Whipple, le syndrome des camps de concentration, le lupus érythémateux aigu disséminé, l'arrêt cardiaque, l'encéphalopathie consécutive au SIDA, l'hypothyroïdie, l'hypohypophysie ou l'alcoolisme chronique.

7. Médicament ou composition pharmaceutique destiné à être utilisé selon la revendication 1 où la détérioration cognitive est une détérioration cognitive atténuée.

8. Médicament ou composition pharmaceutique destiné à être utilisé selon la revendication 1 qui comprend en outre au moins un médicament supplémentaire pour prévenir et/ou traiter une détérioration cognitive sous forme d'une formulation unique.

9. Médicament ou composition pharmaceutique destiné à être utilisé selon la revendication 8 où le médicament supplémentaire pour prévenir et/ou traiter une détérioration cognitive est choisi dans le groupe consistant en les inhibiteurs d'acétylcholinestérase, les antagonistes de récepteur NMDA, les inhibiteurs sélectifs de cyclooxygénase-2, les formulations d'immunoglobulines humaines intraveineuses, les antagonistes de récepteur 5-HT6, les antagonistes de récepteur LTB4, les anticorps monoclonaux humains et les inhibiteurs de production d'amyloïde β.

10. Médicament ou composition pharmaceutique destiné à être utilisé dans la prévention et/ou le traitement d'une détérioration cognitive selon la revendication 1 qui comprend au moins un médicament supplémentaire pour prévenir et/ou traiter une détérioration cognitive.

11. Médicament ou composition pharmaceutique destiné à être utilisé dans la prévention et/ou le traitement d'une détérioration cognitive selon la revendication 10 où le médicament supplémentaire pour prévenir et/ou traiter une détérioration cognitive est choisi dans le groupe consistant en les inhibiteurs d'acétylcholinestérase, les antagonistes de récepteur NMDA, les inhibiteurs sélectifs de cyclooxygénase-2, les formulations d'immunoglobulines humaines intraveineuses, les antagonistes de récepteur 5-HT6, les antagonistes de récepteur LTB4, les anticorps monoclonaux humains et les inhibiteurs de production d'amyloïde β.

12. Médicament ou composition pharmaceutique destiné à être utilisé dans la prévention et/ou le traitement d'une détérioration cognitive selon la revendication 10 où l'acide 2-méthyl-2-[(4-{(1E)-3-[2-(4-méthylbenzoyl)-1H-pyrrol-1-yl]prop-1-én-1-yl}benzyl)oxy]propionique ou un sel ou solvate pharmaceutiquement acceptable de celui-ci est administré simultanément ou successivement avec le médicament supplémentaire pour prévenir et/ou traiter une détérioration cognitive.
